# EUROPEAN PATENT APPLICATION

(11) **EP 4 002 232 A1**
(43) Date of publication of application: **25.05.2022**
(21) Application number: 20841123.1
(22) Date of filing: 22.06.2020
(51) Int. Cl.: G06N 20/20

(54) **DATA ANALYSIS DEVICE, DATA ANALYSIS METHOD, AND DATA ANALYSIS PROGRAM**

(30) Priority: 18.07.2019 JP 2019132543
(71) Applicant: Hitachi High-Tech Corporation, Minato-ku Tokyo 105-6409 (JP)
(72) Inventor: FUKUI, Daisuke, Tokyo 100-8280 (JP); NAKAGAWA, Hiromitsu, Tokyo 100-8280 (JP); TANAKA, Takeshi, Tokyo 100-8280 (JP); SANO, Yuko, Tokyo 100-8280 (JP); MIYAKE, Masatoshi, Tokyo 105-6409 (JP); HORIKOSHI, Nobuya, Tokyo 105-6409 (JP)
(74) Representative: Mewburn Ellis LLP
(86) International application number: PCT/JP2020/024353
(87) International publication number: WO 2021/010093

(57) **Abstract**

An object of the invention is to harmonize prediction accuracy and an analysis time of an ensemble model. Therefore, when performing data analysis using an ensemble model 300 that makes an inference by integrating inferences by first to n-th models, an i-th model (1 ≤ i ≤ n) constituting the ensemble model 300 is selected from an i-th model group of the model data, at least one model group of the first to n-th model groups includes a plurality of models, and the first to n-th models capable of constituting an ensemble model satisfying a performance requirement for data analysis and a constraint requirement for time required for the data analysis are selected from the first to n-th model groups 301 to 303.

## Description

### Technical Field

The present invention relates to a data analysis apparatus, a data analysis method, and a data analysis program.

### Background Art

In order for a person to freely move his/her body, locomotive organs made up of bones, joints, muscles and nerves need to function normally. Locomotive syndrome ("locomo") refers to a condition in which one or more locomotive organs are impaired and movement functions such as standing, walking, running, and sitting are declined. When such a decline in the movement functions progresses, a trouble occurs even in a daily life. It is said that locomotor disorders that require a hospital treatment usually occur after an age of 50, and locomotor disorders in the elder lead to a risk of needing support or care. Since the locomotor disorders progress gradually, a need for prevention, early detection, and appropriate coping of the locomo is recognized. Patent Literature 1 discloses a walking mode analysis apparatus that measures a walking state of a measurement subject, calculates feature amount data from a measurement result, and analyzes a walking mode of the measurement subject using calculated feature amount data and an analysis model.

In Patent Literature 2, in constructing a prediction model, candidates for preprocessing of input data, a data learning method based on a hyperparameter, and the like are set in advance, and a pipeline capable of constructing a prediction model with higher prediction accuracy is selected from combinations (referred to as pipelines) of these candidates. A search is performed using sample data extracted at a predetermined ratio from learning data so that time required for a search for the pipeline does not increase even when the number of candidates increases, the extraction ratio of the sample data is increased as long as processing time does not exceed a time limit, and a combination in which the prediction accuracy of the prediction model is high is searched for.

### Citation list

### Patent Literature

PTL 1: Japanese Patent No. 6509406
PTL 2: JP-A-2018-190130

### Summary of Invention

### Technical Problem

A decline in movement functions of a person is represented as a gait disorder. It is effective to know a walking state of the person, which promotes early detection and remission of the locomo, and to inform a subject in an easy-to-understand manner. From a viewpoint of prevention or early detection of a locomotor disorder, it is desirable that the analysis apparatus as disclosed in Patent Literature 1 is provided not only in a medical institution but also in a fitness gym or the like, and even a measurement subject who is unaware of the locomotor disorder can easily be aware of his/her walking state.

However, the more precisely and accurately a walking mode is analyzed, the more enormous the number of feature amount data used for analysis is, and time required for calculation of the feature amount data and the analysis using the feature amount data also increases. When it takes a long waiting time to obtain an analysis result, the waiting time may be avoided by the measurement subject who is unaware of a locomotor disorder. In particular, when the analysis apparatus is provided in a place close to the measurement subject, it is desirable to calculate the feature amount data and analyze the walking state by a personal computer (PC) or the like that is generally used, and it cannot be assumed that a computer with particularly high computing capability is used.

Patent Literature 2 discloses shortening a search time for pipeline selection for construction of a prediction model, and does not refer to time required for analysis using the prediction model.

### Solution to Problem

A data analysis apparatus according to an embodiment of the invention performs data analysis using an ensemble model that makes an inference by integrating inferences by first to n-th models. The data analysis apparatus includes: a processor; a memory; a storage; and a data analysis program read into the memory and executed by the processor. The storage stores model data in which first to n-th model groups each including one or more models are registered, an i-th model (1 ≤ i ≤ n) constituting the ensemble model is selected from an i-th model group of the model data, at least one model group of the first to n-th model groups includes a plurality of models, and the data analysis program includes: an ensemble model creation processing unit configured to present, from the respective first to n-th model groups, options of the first to n-th models capable of constituting the ensemble model satisfying a performance requirement for data analysis and a constraint requirement for time required for the data analysis; and an ensemble analysis processing unit configured to receive selection of the presented options of the first to n-th models and make an inference by the ensemble model using the selected first to n-th models.

### Advantageous Effect

In an analysis using an ensemble model, prediction accuracy and an analysis time of the ensemble model are harmonized.

Other technical problems and novel characteristics will be apparent from the description and the accompanying drawings.

### Brief Description of Drawings

[FIG. 1] FIG. 1 shows a hardware configuration of a data analysis system.
[FIG. 2] FIG. 2 shows a software configuration of the data analysis system.
[FIG. 3] FIG. 3 is a schematic diagram of an ensemble model.
[FIG. 4] FIG. 4 shows an example of analysis setting data.
[FIG. 5] FIG. 5 shows an example of domain knowledge data.
[FIG. 6] FIG. 6 shows a processing flow for analyzing a walking mode of a measurement subject.
[FIG. 7] FIG. 7 shows a data structure of measurement data.
[FIG. 8] FIG. 8 shows a data structure of feature amount data.
[FIG. 9] FIG. 9 shows a data structure of prediction result data.
[FIG. 10] FIG. 10 shows an evaluation flow of a model (weak recognizer).
[FIG. 11] FIG. 11 shows a data structure of model data.
[FIG. 12] FIG. 12 shows a flow for selecting a model (weak recognizer) and selecting a feature amount to be calculated.
[FIG. 13] FIG. 13 shows an ensemble model determination flow.
[FIG. 14] FIG. 14 shows a data structure of ensemble model data.
[FIG. 15] FIG. 15 shows a data structure of selected feature amount data.

### Description of Embodiments

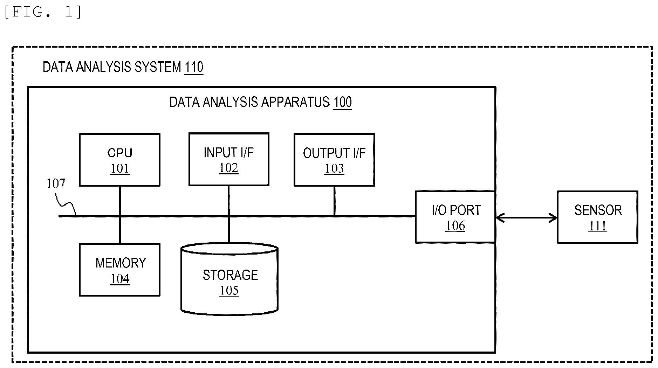

FIG. 1 shows a hardware configuration of a data analysis system 110 that analyzes a walking mode of a pedestrian. The data analysis system 110 includes a sensor 111 that measures walking of a measurement subject, and a data analysis apparatus 100 that measures the walking of the measurement subject using the sensor 111 and analyzes a walking mode from a measurement result.

The data analysis apparatus 100 includes a central processing unit (CPU) 101, an input interface (I/F) 102, an output I/F 103, a memory 104, a storage 105, and an I/O port 106, which are connected by an internal bus 107. The data analysis apparatus 100 is an information processing apparatus that can be implemented by a general-purpose computer. The input I/F 102 is connected to an input device such as a keyboard or a mouse, and the output I/F 103 is connected to a display or a printer to implement a graphical user interface (GUI) for an operator. The storage 105 usually includes a nonvolatile memory such as a HDD, a SSD, a ROM, or a flash memory, and stores a program to be executed by the data analysis apparatus 100, data to be processed by the program, and the like. The memory 104 includes a random access memory (RAM), and temporarily stores the program, data necessary for executing the program, and the like according to a command of the CPU 101. The CPU 101 executes the program loaded from the storage 105 to the memory 104.

The data analysis apparatus 100 issues a collection command of sensing data to the sensor 111. The sensor 111 senses the walking of the measurement subject in response to the command and transmits a measurement result to the data analysis apparatus 100. A distance sensor based on a time of flight (TOF) method can be used as the sensor 111. In order to capture the walking mode of the measurement subject, it is necessary to measure a movement (trajectory) in a three-dimensional space of a measurement point (joint or the like) of a body of the measurement subject during walking, and the distance sensor has an advantage that coordinates of the measurement point in the three-dimensional space can be directly obtained. The sensor 111 is not limited to the distance sensor and may be a video camera and perform an image analysis from a video obtained by imaging a measurer during walking by the video camera. A sensor such as an acceleration sensor, an angle sensor, or a gyro sensor may be used. It is also possible to use a plurality of types of sensors.

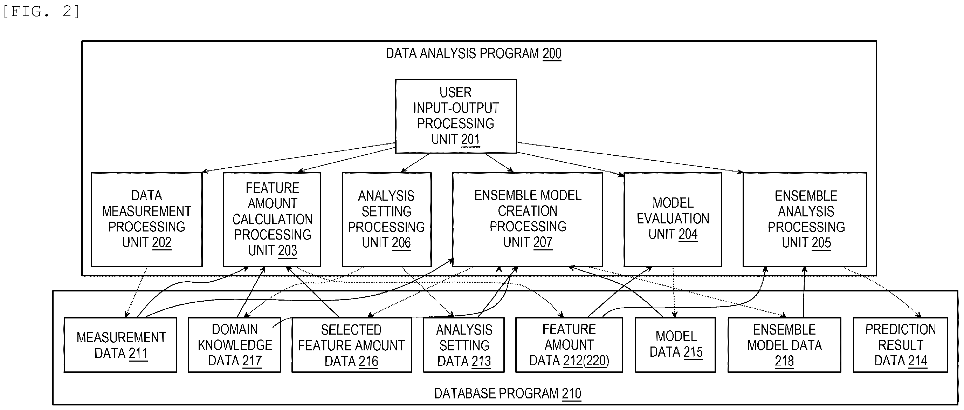

FIG. 2 shows a software configuration of the data analysis system 110, and shows programs executed in the data analysis apparatus 100 and a relation between the programs. A data analysis program 200 has a function of measuring walking and analyzing a walking mode from a measurement result. A user input-output processing unit 201 is an interface program by which an operator inputs instructions and information to modules 202 to 207. The modules 202 to 207 are programs that execute functions related to measurement of walking or analysis of a walking mode, and contents thereof will be described later. A database program 210 has a function of storing and managing measurement data or an analysis model necessary for the data analysis system 110 in the storage 105.

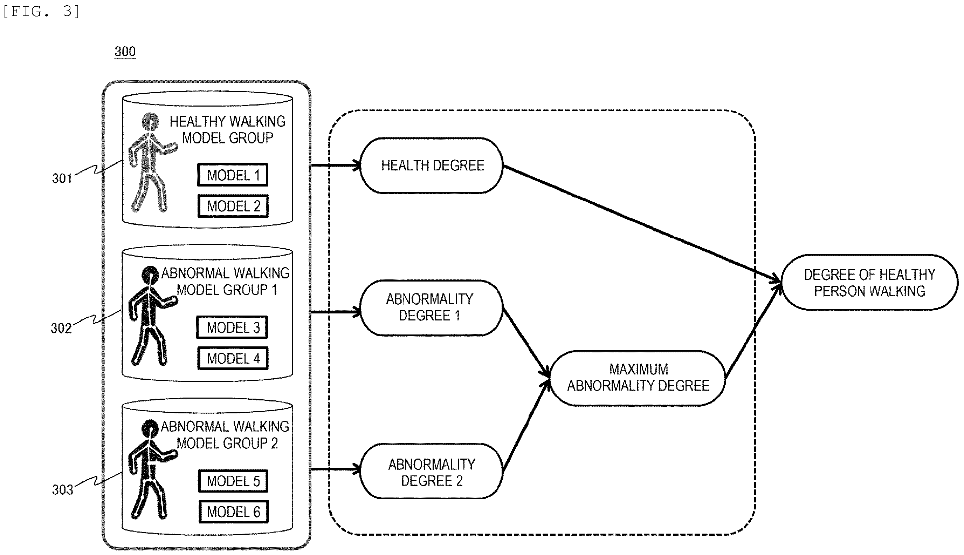

In the present embodiment, the walking mode is analyzed using an ensemble model. The ensemble model is a model that integrates inferences by a plurality of models (weak recognizers) into one inference. FIG. 3 is a schematic diagram of an ensemble model applied to the present embodiment. An ensemble model 300 integrates determination results of three models (weak recognizers) and determines whether the walking of the measurement subject is healthy. The three models includes a healthy walking model that determines whether the walking of the measurement subject is healthy walking, a first abnormal walking model that determines whether the walking of the measurement subject is abnormal walking 1, and a second abnormal walking model that determines whether the walking of the measurement subject is abnormal walking 2. Each of the abnormal walking 1 and 2 is a specific walking state that is regarded as a gait disorder. The ensemble model 300 compares an abnormality degree 1 (probability of the abnormal walking 1) output from the first abnormal walking model and an abnormality degree 2 (probability of the abnormal walking 2) output from the second abnormal walking model, sets a larger one thereof as a maximum abnormality degree (probability of abnormal walking), and integrates the maximum abnormality degree and a health degree (probability of healthy walking) output from the healthy walking model to output a degree of healthy person walking (probability of healthy walking). The plurality of models (weak recognizers) and an integration method thereof shown in FIG. 3 are one example.

It is assumed that the models that constitute the ensemble model 300 and output the healthy degree, the abnormality degree 1, and the abnormality degree 2 are selected from respective model groups, and at least one of the model groups includes a plurality of models. In the example of FIG. 3, the model that outputs the health degree can be selected from models 1 and 2 registered as a healthy walking model group 301, the model that outputs the abnormality degree 1 can be selected from models 3 and 4 registered as a first abnormal walking model group 302, and the model that outputs the abnormality degree 2 can be selected from models 5 and 6 registered as a second abnormal walking model group 303. The data analysis system 110 of the present embodiment selects one model from the models registered in the model groups 301 to 303 in accordance with performance and constraints required by data analysis, thereby implementing analysis according to needs of the measurement subject.

Accordingly, in order to adapt the data analysis system 110 to a measurement subject group having different performances required by data analysis and different constraints allowed by data analysis, an administrator of the data analysis system 110 activates the analysis setting processing unit 206 and registers analysis setting data 213 and domain knowledge data 217 (see FIG. 2).

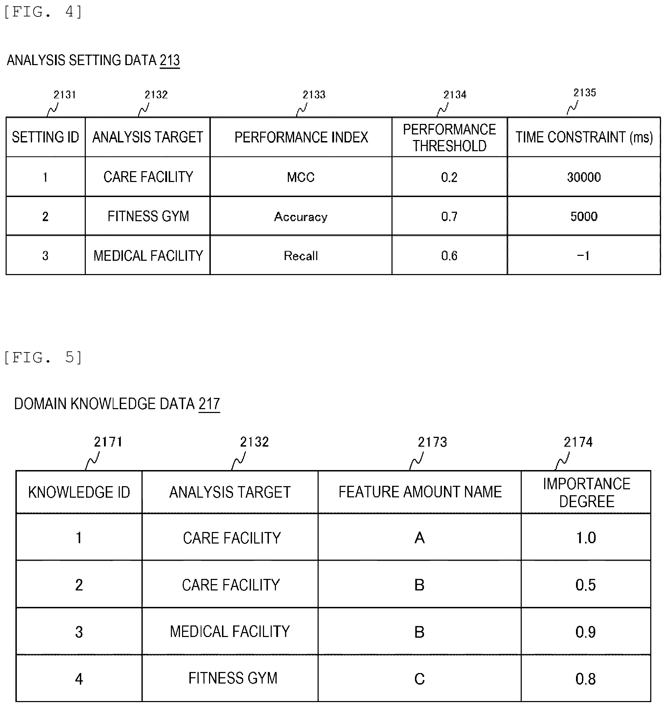

FIG. 4 shows an example of the analysis setting data 213. The analysis setting data 213 defines the performance and constraints of the ensemble model applied to each measurement subject group. An analysis target 2132 indicates the measurement subject group, and the measurement subject group is defined depending on a place where the system 110 is used. A definition method is not limited to this example and is freely selected. A performance requirement and a constraint requirement of the ensemble model are defined for each analysis target. The performance requirement is defined by a performance index 2133 and a performance threshold 2134. For example, in a case of a measurement subject group (care facility) defined as setting ID 1, the performance requirement is that the performance index Matthew correlation coefficient (MCC) is 0.2 or more. When defining the performance requirement, it is expected to obtain a result relatively suitable for needs of each measurement subject group by using a performance index selected from a plurality of performance indexes. For example, an index reflecting a required performance and a threshold for the index are set depending on whether the measurement subject group emphasizes accuracy or reproducibility. On the other hand, the constraint requirement is defined by a time constraint 2135 indicating an upper limit of time allowed for data analysis. In this example, a measurement subject (setting ID 2) of a fitness gym is subject to a strict constraint on an analysis time, and a measurement subject (setting ID 3) of a medical facility has no constraint on the analysis time (setting the time constraint 2135 to a negative value indicates that no constraint is set).

FIG. 5 shows an example of the domain knowledge data 217. The domain knowledge data 217 defines an important feature amount for each measurement subject group. The feature amount is a feature amount calculated based on a movement (trajectory), which is measured by the sensor 111, in a three-dimensional space of a measurement point (joint or the like) of a body of the measurement subject during walking, and is a movement, a correlation, or the like of a joint or an axis of the measurement subject during walking. The domain knowledge data 217 is feature amount data included in the analysis regardless of a weighting in a prediction model. For example, feature amount data that a doctor or trainer wants to refer to when explaining an analysis result to the measurement subject is applicable. The domain knowledge data 217 is also defined for each measurement subject group having the same definition as the analysis setting data 213. In this example, an importance degree 2174 is defined for a feature amount registered in a feature amount name 2173. For example, in the analysis target "care facility", a feature amount A has a higher importance degree than a feature amount B (knowledge IDs 1 and 2). When measured feature amounts are narrowed, it is possible to exclude an analysis target having a small value of importance degree from calculation targets. In this example, the importance degree of each feature amount is defined, and ranking of the importance degree of each feature amount for each measurement subject group may be defined.

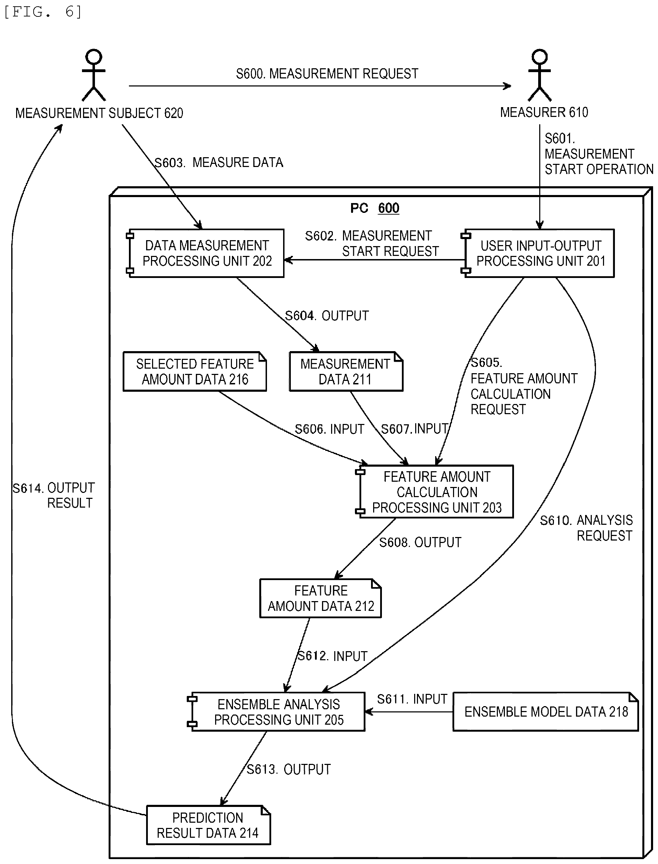

A processing flow in which a measurer 610 analyzes a walking mode of a measurement subject 620 by a PC 600 that is the data analysis apparatus 100 will be described with reference to FIG. 6. The PC 600 is disposed in a specific place (a care facility, a fitness gym, a medical facility, or the like) defined as an analysis target. In the PC 600, an ensemble model is constructed to satisfy performance requirements and constraint requirements set for arrangement locations defined in the analysis setting data 213 described above, and feature amount data necessary for the constructed ensemble model is selected.

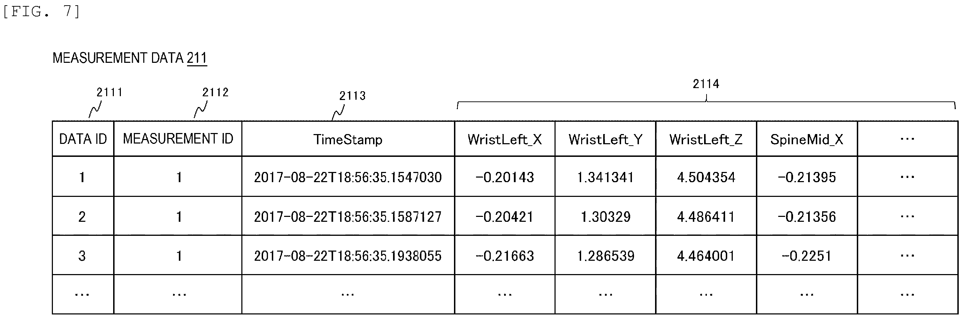

When the measurement subject 620 makes a measurement request to the measurer 610 (S600), the measurer 610 performs a measurement start operation on the user input-output processing unit 201 (S601). First, the user input-output processing unit 201 issues a measurement start request to the data measurement processing unit 202 (S602). The data measurement processing unit 202 measures the walking of the measurement subject 620 using the sensor 111 (S603), and stores obtained measurement data in the storage 105 (S604) . FIG. 7 shows a data structure of measurement data 211.

The measurement data 211 is a trajectory of a measurement point of the measurement subject in the three-dimensional space, and (X, Y, Z) coordinates 2114 of each measurement point for each time indicated by a time stamp 2113 are stored. As the measurement point, a joint or the like that affects the walking mode is set. A data ID 2111 is an ID assigned to each record included in the measurement data 211, and a measurement ID 2112 is an ID assigned to each measurement request of the measurement subject 620.

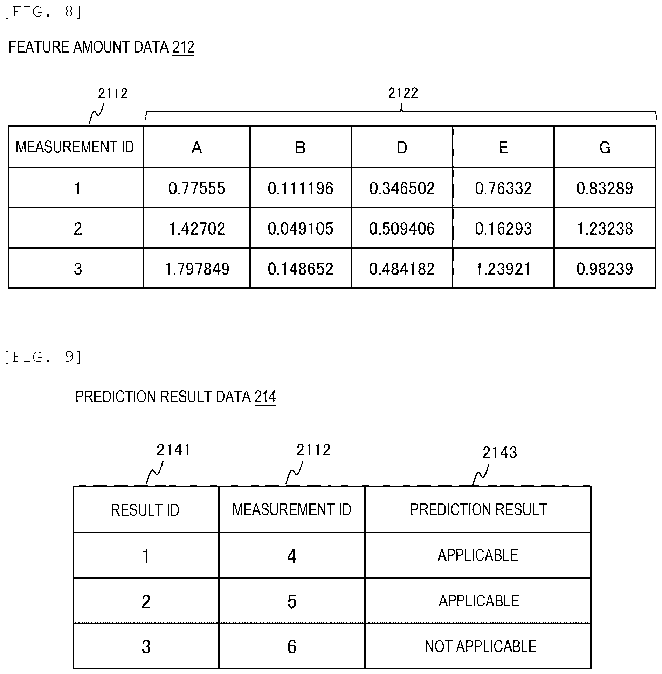

When the measurement of the walking of the measurement subject 620 ends, the user input-output processing unit 201 issues a feature amount calculation request to the feature amount calculation processing unit 203 (S605). The feature amount calculation processing unit 203 receives inputs of selected feature amount data 216 for specifying a feature amount to be used for the ensemble model and the measurement data 211 of the measurement subject 620 (S606, 607), calculates feature amount data 212 specified by the selected feature amount data 216, and stores the obtained feature amount data in the storage 105 (S608). FIG. 8 shows a data structure of the feature amount data 212. In the feature amount data 212, a feature amount 2122 specified by the selected feature amount data 216 is stored for each measurement ID 2112.

When the calculation of the feature amount selected by the selected feature amount data 216 ends, the user input-output processing unit 201 issues an analysis request to the ensemble analysis processing unit 205 (S610). The ensemble analysis processing unit 205 receives inputs of ensemble model data 218 and the feature amount data 212 (S611, S612), performs analysis using the ensemble model, stores prediction result data 214 (for example, in the example of FIG. 3, a degree of healthy person walking or a determination result of whether walking based on the degree of healthy person walking is healthy) in the storage 105 (S613), and presents a result to the measurement subject 620 by displaying the result on a display or the like (S614) . FIG. 9 shows a data structure of the prediction result data 214. In the prediction result data 214, a prediction result 2143 for each measurement ID 2112 is stored.

FIG. 6 shows a processing flow in which the PC 600 analyzes the walking mode under predetermined performance requirements and constraint requirements. For example, a time zone in which there is no constraint such as off-business hours may be set in advance, a feature amount not designated in the selected feature amount data 216 may be calculated in the time zone, and analysis may be performed by the ensemble model using different models (weak recognizers). Alternatively, the measurement data 211 may be transferred to another data analysis apparatus 100, and the walking mode may be analyzed without a constraint. Further, when the measurer 610 can diagnose the walking mode of the measurement subject 620, a diagnosis result of the walking mode of the measurement subject 620 diagnosed by the measurer 610 is tagged as teacher data to the measurement data 211 or all feature amount data calculated from the measurement data 211. Accordingly, the measurement data of the measurement subject can be used as learning data for model relearning.

Before executing the processing flow of FIG. 6, the construction of the ensemble model and the selection of the feature amount to be calculated are performed to satisfy the definition of the analysis setting data 213. Hereinafter, the procedure will be described.

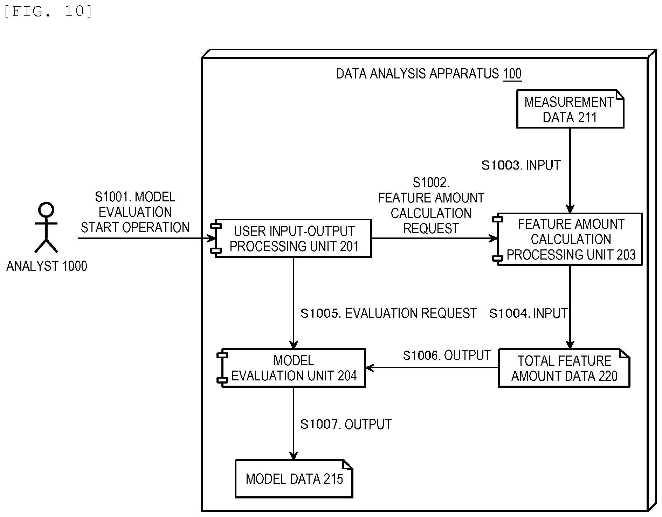

FIG. 10 shows an evaluation flow of models (weak recognizers) constituting the ensemble model. In the case of the ensemble model 300 shown in FIG. 3, the evaluation flow shown in FIG. 10 is executed for each of the models 1 to 6 included in the healthy walking model group 301, the first abnormal walking model group 302, and the second abnormal walking model group 303. This evaluation flow is performed each time learning is performed on each model. For example, each time the data analysis apparatus 100 learns a model, it is desirable to execute the evaluation flow of FIG. 10 and store an evaluation result together with the model.

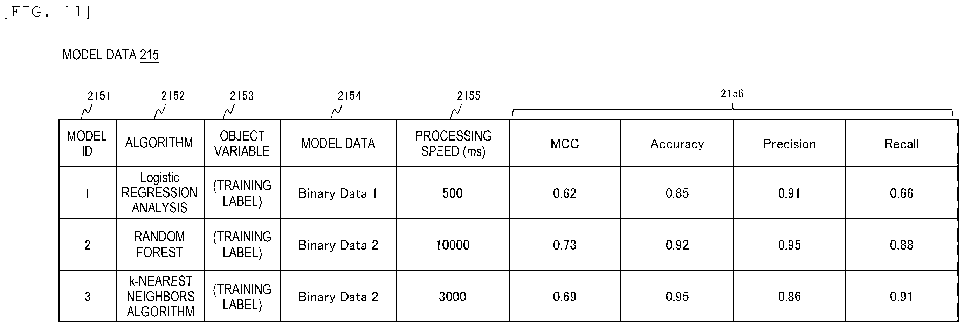

An analyst 1000 performs a model evaluation start operation on the user input-output processing unit 201 (S1001). First, the user input-output processing unit 201 issues a feature amount calculation request to the feature amount calculation processing unit 203 (S1002). The feature amount calculation processing unit 203 receives inputs of the measurement data 211 stored in the storage 105 (S1003), and calculates total feature amount data 220 (S1004). Any measurement data may be used as the measurement data 211, and for example, measurement data used for learning a model may be used. The total feature amount data 220 includes all feature amounts used by a model (weak recognizer) that is an option of the ensemble model to be evaluated. When the total feature amount data 220 is calculated, the user input-output processing unit 201 issues a model evaluation request to the model evaluation unit 204 (S1005). The model evaluation unit 204 receives an input of the total feature amount data 220 (S1006), executes evaluation of each model, and stores model data 215 including an evaluation result in the storage 105 (S1007). FIG. 11 shows a data structure of the model data 215.

A model ID 2151 is an ID for specifying each of the models (weak recognizers) constituting the ensemble model. An algorithm used in each model is stored in an algorithm 2152, an object variable (for example, healthy walking, abnormal walking 1, and abnormal walking 2 in the example of FIG. 3) of the model is stored in an object variable 2153, and binary data of the model is stored in model data 2154. Results evaluated by the model evaluation unit 204 are stored in a processing speed 2155 and a performance index 2156. The processing speed 2155 indicates time from when a feature amount is input to each model to when a recognition result is output. The performance index 2156 stores an evaluation result for each performance index (performance index appearing in the analysis setting data 213) used to define the performance requirement of the ensemble model.

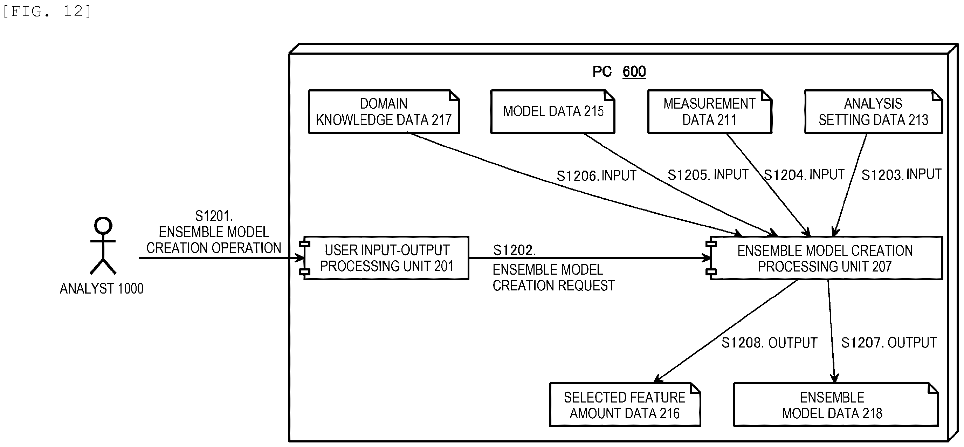

FIG. 12 shows a flow for selecting a model (weak recognizer) to be used for the ensemble model and selecting a feature amount to be calculated. The flow of FIG. 12 is preferably performed by an information processing apparatus that performs actual analysis, that is, the PC 600 that executes the processing flow of FIG. 6 in the present embodiment. The time required for calculating the feature amount and recognizing by the model differs depending on calculation performance and a state of the information processing apparatus. Therefore, it is possible to improve reliability of pre-evaluation results of the performance and constraints of the ensemble model by constructing the ensemble model and selecting the feature amount to be calculated with the information processing apparatus that performs the actual analysis.

The analyst 1000 performs an ensemble model creation operation on the user input-output processing unit 201 (S1201). The user input-output processing unit 201 issues an ensemble model creation request to the ensemble model creation processing unit 207 (S1202). The ensemble model creation processing unit 207 receives inputs of the analysis setting data 213, the measurement data 211, the model data 215, and the domain knowledge data 217 stored in the storage 105 (S1203 to S1206), creates the ensemble model data 218 for specifying the models constituting the ensemble model satisfying the predetermined performance requirements and constraint requirements and the selected feature amount data 216 for specifying a feature amount required to be calculated for the ensemble model, and stores the ensemble model data 218 and the selected feature amount data 216 in the storage 105 (S1207 to S1208).

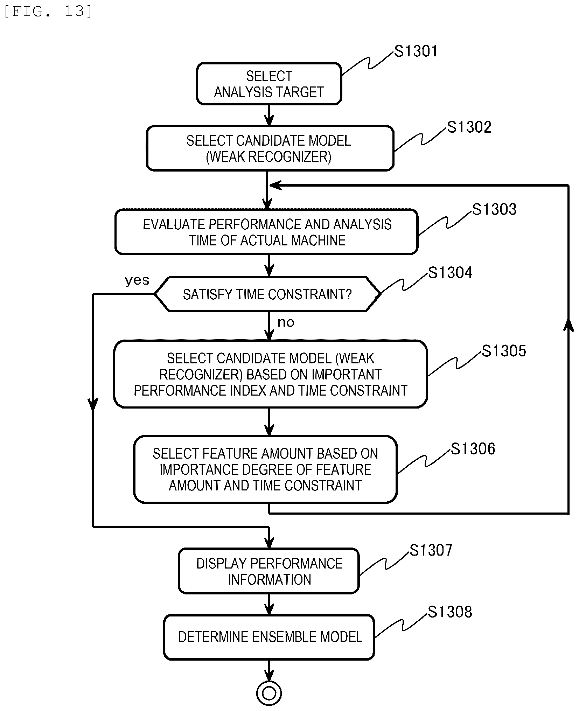

FIG. 13 shows an ensemble model determination flow executed by the ensemble model creation processing unit 207. First, selection of an analysis target (measurement subject group) using the PC 600 is received (S1301). By collating the input analysis target (measurement subject group) with the analysis setting data 213, it is possible to obtain the performance requirements and the constraint requirements required for the ensemble model.

Subsequently, a candidate model (weak recognizer) to be used for the ensemble model is selected (S1302). The candidate model to be used for the ensemble model is selected based on the processing speed 2155 and the performance index 2156 stored in the model data 215. In the selection, a candidate model is selected so that a performance index specified as a performance requirement is highest. In this case, a plurality of candidates may be selected.

Subsequently, for the ensemble model to which the selected candidate model (weak recognizer) is applied, performance and an analysis time of an actual machine are evaluated (S1303). In performance evaluation, the performance index specified as the performance requirement is calculated. The evaluated analysis time includes time required to calculate the feature amount data from the measurement data and time required to perform analysis by the ensemble model from the feature amount data. A calculation time of the feature amount data is time required to calculate a feature amount necessary for analysis by the ensemble model constituted by the candidate model. Since the processing speed 2155 stored in the model data 215 is not limited to the processing speed evaluated by the PC 600, it is possible to estimate a more accurate time required for the analysis by the ensemble model by the PC 600 performing the analysis from the actual measurement data 211. The measurement data used for an analysis time evaluation may be the measurement data used for learning the model, measurement data measured by the PC 600 in the past, or any measurement data.

When the analysis time evaluation by the actual machine (S1303) satisfies the time constraint 2135 (see FIG. 4) of the analysis target (yes in S1304), performance information on the ensemble model using the selected model (weak recognizer) is displayed on a display or the like (S1307). When there are a plurality of candidates, each candidate is displayed together with the performance information. The analyst 1000 checks the performance information and determines a model (weak recognizer) to be used for the ensemble model from the presented model candidates (S1308).

When the analysis time evaluation by the actual machine (S1303) does not satisfy the time constraint 2135 of the analysis target (no in S1304), a model candidate is selected so that the performance index specified as the performance requirement is as high as possible based on a deviation between the performance index specified as the performance requirement, the analysis time evaluated in S1303, and the time constraint as the constraint requirement (S1305).

At this time, a model candidate is selected so that the feature amount to be calculated is limited based on the deviation between the importance degree of the feature amount, the analysis time evaluated in S1303, and the time constraint that is the constraint requirement (S1306). As the importance degree of the feature amount, both an importance degree in an analysis algorithm and an importance degree in a description of an analysis result to the measurement subject are considered. The importance degree in an analysis algorithm can be determined from the binary data of the model data 2154, and the importance degree in a description of an analysis result to the measurement subject can be determined from the domain knowledge data 217. Regarding at least one model constituting the ensemble model, by omitting the calculation of the feature amount having a small influence on the analysis result or the description thereof (this state is referred to as an "input constrained state"), it is possible to expect that the time required for the calculation of the feature amount is reduced while preventing the decline of the performance as much as possible. Also in S1305 and S1306, a plurality of candidates may be selected.

The performance and the analysis time of the actual machine are evaluated again (S1303) based on the selected model candidate and a feature amount candidate, and the selection and the ensemble model evaluation by the actual machine are repeated while changing a combination of models (weak recognizers) constituting the ensemble model and the selection of the feature amount until the model candidate and the feature amount candidate satisfying the time constraint are obtained.

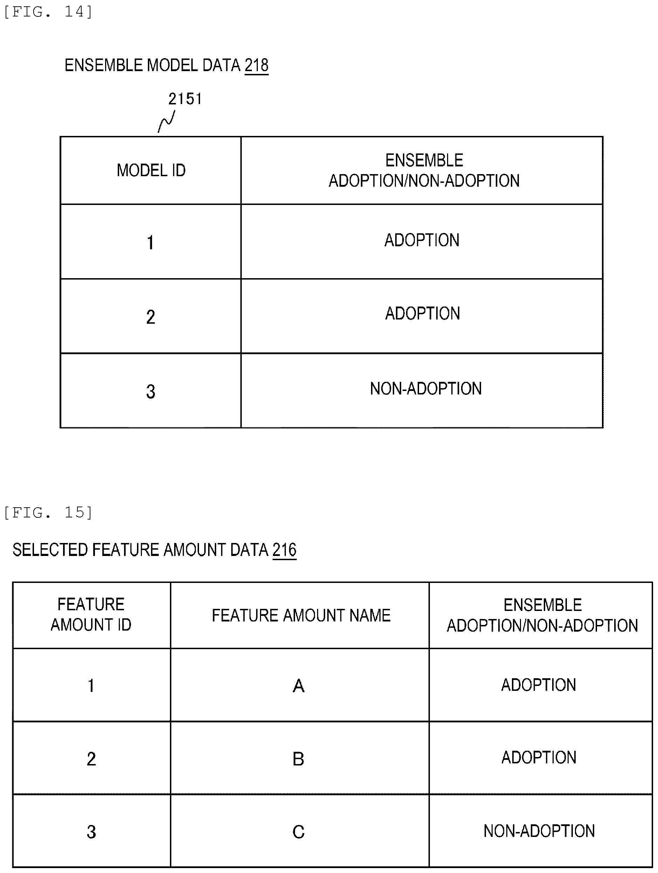

FIG. 14 shows a data structure of the ensemble model data 218 output by the ensemble model creation processing unit 207. For each model (weak recognizer) registered as the model data 215, adoption/non-adoption for the ensemble model is registered.

FIG. 15 shows a data structure of the selected feature amount data 216 output by the ensemble model creation processing unit 207. For each of the feature amounts that can be calculated by the data analysis apparatus 100, adoption/non-adoption for the ensemble model is registered.

While the invention made by the present inventor has been specifically described based on the embodiment, the invention is not limited thereto, and various modifications may be made without departing from the scope of the invention. In the embodiment, a walking mode analysis apparatus that analyzes the walking mode of the measurement subject has been described as an example, and the invention is widely applicable to an apparatus, a system, a method, and a program that perform data analysis using an ensemble model.

### Reference Sign List

- 100: data analysis apparatus
- 101: CPU
- 102: input I/F
- 103: output I/F
- 104: memory
- 105: storage
- 106: I/O port
- 107: internal bus
- 110: data analysis system
- 111: sensor
- 200: data analysis program
- 201: user input-output processing unit
- 202: data measurement processing unit
- 203: feature amount calculation processing unit
- 204: model evaluation unit
- 205: ensemble analysis processing unit
- 206: analysis setting processing unit
- 207: ensemble model creation processing unit
- 210: database program
- 211: measurement data
- 212: feature amount data
- 213: analysis setting data
- 214: prediction result data
- 215: model data
- 216: selected feature amount data
- 217: domain knowledge data
- 218: ensemble model data
- 220: total feature amount data
- 300: ensemble model
- 301: healthy walking model group
- 302: first abnormal walking model group
- 303: second abnormal walking model group

## Claims

1. A data analysis apparatus that performs data analysis using an ensemble model that makes an inference by integrating inferences by first to n-th models, the data analysis apparatus comprising:
a processor;
a memory;
a storage; and
a data analysis program read into the memory and executed by the processor, wherein
the storage stores model data in which first to n-th model groups each including one or more models are registered,
an i-th model (1 ≤ i ≤ n) constituting the ensemble model is selected from an i-th model group of the model data,
at least one model group of the first to n-th model groups includes a plurality of models, and
the data analysis program includes:
an ensemble model creation processing unit configured to present, from the respective first to n-th model groups, options of the first to n-th models capable of constituting an ensemble model satisfying a performance requirement for data analysis and a constraint requirement for time required for the data analysis; and
an ensemble analysis processing unit configured to receive selection of the presented options of the first to n-th models and make an inference by the ensemble model using the selected first to n-th models.

2. The data analysis apparatus according to claim 1, wherein
the storage stores analysis setting data for setting the performance requirement and the constraint requirement for each of a plurality of analysis targets, and
the ensemble model creation processing unit presents the options of the first to n-th models capable of constituting the ensemble model satisfying the performance requirement and the constraint requirement of an analysis target that is a target of the data analysis among the plurality of analysis targets.

3. The data analysis apparatus according to claim 2, wherein
the performance requirement is defined by a performance index and a threshold of the performance index, and
an index corresponding to the plurality of analysis targets is set as the performance index.

4. The data analysis apparatus according to claim 2, wherein
the constraint requirement is provided as an upper limit of an analysis time including time required to calculate feature amount data from measurement data and time required to perform the data analysis using the ensemble model from the feature amount data.

5. The data analysis apparatus according to claim 4, wherein
the ensemble model creation processing unit makes an inference in an input constrained state in which a feature amount input to at least one model is selected among the presented options of the first to n-th models, and presents the options of the first to n-th models capable of constituting the ensemble model satisfying the performance requirement and the constraint requirement in the input constrained state.

6. The data analysis apparatus according to claim 5, wherein
the storage has domain knowledge data indicating importance of a feature amount in the analysis target that is a target of the data analysis, and
the ensemble model creation processing unit selects a feature amount input to an ensemble model in the input constrained state based on the domain knowledge data and the importance of the feature amount in a model.

7. The data analysis apparatus according to claim 5, wherein
the ensemble model creation processing unit receives selection of the options of the presented first to n-th models, and stores, in the storage, ensemble model data for specifying the first to n-th models used in an ensemble model used by the ensemble analysis processing unit, and selected feature amount data for specifying a feature amount selected as a feature amount input to the ensemble model used by the ensemble analysis processing unit.

8. The data analysis apparatus according to claim 7, wherein
the data analysis program further includes a feature amount calculation processing unit configured to calculate feature amount data from measurement data,
the feature amount calculation processing unit calculates the feature amount data from the measurement data for a feature amount specified in the selected feature amount data, and
the ensemble analysis processing unit makes an inference by inputting the feature amount data calculated by the feature amount calculation processing unit to the ensemble model using the first to n-th models specified in the ensemble model data.

9. The data analysis apparatus according to claim 8, wherein
the feature amount calculation processing unit calculates the feature amount data from the measurement data for a feature amount not specified in the selected feature amount data in a predetermined time zone.

10. The data analysis apparatus according to claim 9, wherein
the feature amount data calculated by the feature amount calculation processing unit from the measurement data is used for learning of a model stored in the storage.

11. A data analysis method for performing data analysis using an ensemble model that makes an inference by integrating inferences by first to n-th models, the data analysis method comprising:
storing in advance model data in which first to n-th model groups each including one or more models are registered, an i-th model (1 ≤ i ≤ n) constituting the ensemble model being selected from an i-th model group of the model data, at least one model group of the first to n-th model groups including a plurality of models;
presenting, from the respective first to n-th model groups, options of the first to n-th models capable of constituting an ensemble model satisfying a performance requirement for the data analysis and a constraint requirement for time required for the data analysis; and
receiving selection of the presented options of the first to n-th models and making an inference by the ensemble model using selected first to n-th models.

12. The data analysis method according to claim 11, further comprising:
making an inference in an input constrained state in which a feature amount input to at least one model is selected among the presented options of the first to n-th models; and
presenting the options of the first to n-th models capable of constituting the ensemble model satisfying the performance requirement and the constraint requirement in the input constrained state.

13. The data analysis method according to claim 12, further comprising:
calculating first feature amount data from measurement data for a first feature amount input to an ensemble model that preforms data analysis; and
making an inference by inputting the calculated first feature amount data to the ensemble model that preforms the data analysis.

14. The data analysis method according to claim 13, further comprising:
calculating second feature amount data from the measurement data for a second feature amount other than the first feature amount in a predetermined time zone.

15. A data analysis program that performs data analysis using an ensemble model that makes an inference by integrating inferences by first to n-th models on an information processing apparatus that stores model data in which first to n-th model groups each including one or more models are registered, wherein
an i-th model (1 ≤ i ≤ n) constituting the ensemble model is selected from an i-th model group of the model data,
at least one model group of the first to n-th model groups includes a plurality of models, and
the data analysis program comprises:
a first step of presenting, from the respective first to n-th model groups, options of the first to n-th models capable of constituting an ensemble model satisfying a performance requirement for the data analysis and a constraint requirement for time required for the data analysis; and
a second step of receiving selection of the presented options of the first to n-th models and making an inference by the ensemble model using selected first to n-th models.
